# EUROPEAN PATENT APPLICATION

(11) **EP 4 506 020 A2**
(43) Date of publication of application: **12.02.2025**
(21) Application number: 23784017.8
(22) Date of filing: 06.04.2023
(51) Int. Cl.: A61L 27/14, A61L 27/36, A61K 35/12, A61K 47/06, A61K 47/30

(54) **ENZYME-FREE PROCESSES TO PRODUCE HYDROGELS**

(30) Priority: 07.04.2022 BR 102022006759
(71) Applicant: Tissuelabs Pesquisa E Desenvolvimento Ltda, 05350-000 São Paulo (BR)
(72) Inventor: LIGUORI, Gabriel Romero, 01417-010 São Paulo (BR); LIGUORI, Tácia Tavares De Aquinas, 01417-010 São Paulo (BR); DE SOUZA, Fernanda Carla Bombaldi, 05577-200 São Paulo (BR); DE SOUZA, Renata Francielle Bombaldi, 05577-200 São Paulo (BR)
(74) Representative: Nederlandsch Octrooibureau
(86) International application number: PCT/BR2023/050115
(87) International publication number: WO 2023/193080

(57) **Abstract**

The present invention is in the field of tissue and organ bioengineering and refers to an enzyme-free process to produce a tissue-specific extracellular matrix solution, based on a decellularized extracellular matrix, comprising: (a) decellularizing a tissue or organ to obtain the isolated extracellular matrix; and (b) hydrolyzing the isolated extracellular matrix in an acidic environment, in the absence of proteolytic enzymes, optionally followed by purification of the solution. Still, the present invention refers to an enzyme-free process to produce a tissue-specific hydrogel. Thus, the present invention also relates to hydrogels produced according to the current processes, and to ancillary inventions, having the tissue-specific extracellular matrix solution, the hydrogel, and/or the products derived from the tissue-specific extracellular matrix solution as the core that connects the inventive aspects disclosed in this document.

## Description

### Field of the invention

The present invention is in the field of bioengineering of tissues and organs. In particular, the present invention primarily relates to an enzyme-free process for obtaining a tissue-specific extracellular matrix solution, which is particularly useful for obtaining tissue-specific hydrogels and products derived from the tissue-specific extracellular matrix solution, which in turn are useful for the reconstitution of tissues and organs, such as for three-dimensional cell culture and its derivations, like bioprinting. Furthermore, the present invention relates to tissue-specific hydrogels and products derived from the tissue-specific extracellular matrix solution, produced according to the current processes, which are free of proteolytic enzymes, and are therefore suitable for human application, such as in tissue or organ transplants.

The present invention also refers to ancillary inventions, having the tissue-specific extracellular matrix solution, the tissue-specific hydrogel, and/or the derived products of the tissue-specific extracellular matrix solution as the core that connects the inventive aspects revealed by this document.

Thus, the present invention also refers to a process for producing a tissue-specific soluble powder from the tissue-specific neutral extracellular matrix solution, from the hydrogel or derivative product of the extracellular matrix solution of the present invention, and to the tissue-specific soluble powder; to a process to produce a tissue-specific membrane from the tissue-specific neutral extracellular matrix solution, from the hydrogel or derived product of the extracellular matrix solution of the present invention, and to the tissue-specific membrane; a process to provide bioactive compounds to regenerate tissue using the tissue-specific neutral extracellular matrix solution, the hydrogel or derivative product of the extracellular matrix solution of the present invention; a process to provide bioactive compounds to regenerate tissue using the tissue-specific membrane of the present invention; a process *in vitro* to provide bioactive compounds by means of the hydrogel or by means of the derivative product of the extracellular matrix solution of this invention; the use of the tissue-specific extracellular matrix solution, of the hydrogel or derived product of the extracellular matrix solution of this invention for the manufacture of a product; other uses of the tissue-specific extracellular matrix solution, of the hydrogel or of the derived product of the extracellular matrix solution of this invention; to an additive ingredient for cell culture medium comprising the tissue-specific soluble powder of this invention; to a process for growing cells within the hydrogel or derivative product of the extracellular matrix solution of this invention; to a process for cultivating cell aggregates within the hydrogel or derivative product of the extracellular matrix solution of this invention; a process for bioprinting 3D bioinks comprising cells or cell aggregates within the tissue-specific neutral extracellular matrix solution, hydrogel, or derivative product of the extracellular matrix solution of the present invention; a process for 3D bioprinting, from the tissue-specific extracellular matrix solution produced by the enzyme-free process to produce such solution of the present invention; and a kit comprising the tissue-specific extracellular matrix solution produced by the enzyme-free process to produce such solution of the present invention, the tissue-specific soluble powder or product derived from the tissue-specific extracellular matrix solution of the present invention.

### Background of the invention

The existing hydrogels in the state of the art are mostly tissue-specific (or organ-specific) hydrogels. In a simplified way, from animal organs, such as the liver, kidney, heart, etc., through a process of cell extraction or decellularization, the extracellular matrix is obtained, composed of proteins that support the cells. This extracellular matrix is used to produce hydrogels that, in turn, are used to reconstruct tissues or organs, such as for three-dimensional (3D) cell culture and its derivations, such as 3D bioprinting, with potential application in transplants.

Many of the state-of-the-art hydrogel production processes involve the enzymatic digestion of extracellular matrix proteins. It happens that, in these processes, these enzymes can remain present in the final products, making them unsuitable for human application, given the potential for reactivation of enzymes in the human body with consequent unwanted proteolysis of the tissues.

Thus, as a solution to the aforementioned problem of the state of the art, the present invention was revealed, which refers to a process for producing a tissue-specific extracellular matrix solution, useful for obtaining hydrogels and products derived from said solution, without the need for proteolytic enzymes, with the products generated from it being suitable for human application, such as in tissue or organ transplants. They may also have other applications and generate other products, such as those revealed in this document, as well as their production processes.

Some documents on the state of the art, such as document PI 0800603-2 and BR 10 2014 030834 2, refer to methods of decellularization of the extracellular matrix for the manufacture of solid matrices (non-gels) to be used as prostheses or biological grafts, with or without cells, aiming at the therapeutic application of decellularized tissues.

Other documents, such as BR 11 2012 028265 4 and BR 11 2014 026090 7, describe the use of biomaterials containing, but not manufactured from, decellularized extracellular matrix for therapeutic application in various areas.

Document PI 1012502-7 describes the process of obtaining pure type I collagen from decellularized extracellular matrix for therapeutic application in dermal lesions or in conjunction with bone ceramics and/or hydroxyapatite for the manufacture of bone implants.

None of these documents describes the fabrication of tissue-specific solutions, products, or hydrogels, obtained from enzyme-free processes, derived from decellularized extracellular matrix for three-dimensional cell culture or its derivatives, such as bioprinting. It is not by chance that the processes described there differ substantially from what is revealed by the present invention.

Some documents describe, in a generic way, the method of production of hydrogels derived from decellularized extracellular matrix, such as US-8691276-B2, CN-104971380-B, CN-104225667-B, and CN-105169483-B, while others describe the manufacture of hydrogels derived from decellularized extracellular matrix from specific organs, such as US-7919121-B2 and KR-101637548-B1 (liver), EP-2349292-B1 (heart), US-9277999-B2 and US-9861662-B2 (osteocartilaginous tissues), US-8637067-B1 (blood vessels), US-10016464-B2 (amniotic membrane), US-9737635-B2 (nervous tissue), and CN-104307046-B (bone marrow).

Other documents describe the application of these hydrogels, alone or in combination with other compounds, in regenerative therapies, i.e., for tissue repair in therapeutic applications, such as EP-2162142-B1, US-10092676-B2, US-9789224-B2, and US-9592256-B2.

In addition, some documents describe the processing of the extracellular matrix for use in different applications and, among the processing methods described, include the manufacture of hydrogels based on the extracellular matrix, such as US-9788821-B2, US-8187619-B2, and US-9938502-B2.

However, none of these documents reveals or suggests an enzyme-free process for producing an extracellular matrix solution useful for the production of products such as tissue-specific hydrogels, based on decellularized extracellular matrices, such as that revealed by the present invention, nor their products and uses. Thus, they do not have the potential for therapeutic use, with a lower risk of rejection, and/or as an experimental or clinical instrument due to the release of useful factors for tissue regeneration, among others. Furthermore, the processes of the state of the art have disadvantages, which are resolved by the present invention. The enzymatic methods of the state of the art generate weaker and slow cross-linking hydrogels, while the enzyme-free method of the present invention generates stronger and faster cross-linking hydrogels.

Likewise, the prior art documents, by not having disclosed or suggested the products, processes, and uses of the present invention, could not have anticipated or suggested the other embodiments as revealed herein.

### Short Description of the Invention

It is, therefore, the object of the present invention, an enzyme-free process to produce a tissue-specific extracellular matrix solution, based on decellularized extracellular matrix, comprising:
(a) decellularize a tissue or organ to obtain the isolated extracellular matrix; and
(b) hydrolyze the isolated extracellular matrix in an acidic environment, in the absence of proteolytic enzymes, optionally followed by purification of the solution.

In a preferential process realization of the present invention, after step (b), proceed with step (c) of neutralizing the pH of the hydrolyzed extracellular matrix solution from step (b) to obtain a tissue-specific neutral extracellular matrix solution.

In another preferential realization of the process of the present invention, after step (c), step (d) of cross-linking the tissue-specific neutral extracellular matrix solution for transition to a hydrogel is proceeded.

A second object of the present invention refers to an enzyme-free process to produce a tissue-specific hydrogel, based on a decellularized extracellular matrix, comprising:
produce a tissue-specific extracellular matrix solution by means of steps (a) and (b) of the enzyme-free process to produce such a solution of the present invention;
(c) neutralize the pH of the hydrolyzed tissue-specific extracellular matrix solution from step (b) to obtain a neutral tissue-specific extracellular matrix solution; and
(d) cross-link the tissue-specific neutral extracellular matrix solution for transition to a hydrogel.

In a preferential embodiment of this, and of the process, the primary object of the present invention, between steps (a) and (b), proceed with the step of freeze-drying the isolated extracellular matrix and pulverizing the lyophilized material to obtain a fine powder of isolated cell matrix.

In another preferential embodiment of the processes of the present invention, between steps (c) and (d), the steps of lyophilizing the hydrolyzed solution of the extracellular matrix, resolubilizing with an aqueous solution, and the pH may or may not be adjusted are carried out; preferably, resolubilization is with an acidic solution, followed by neutralization and salt balance.

In yet another preferred embodiment of the processes of the present invention, decellularization is performed with at least one detergent selected from the group consisting of Triton X-100, sodium dodecyl sulfate (SDS), sodium lauryl ether sulfate (SLES), sodium cholate, sodium deoxycholate, sulfobetaine-10 (SB-10), sulfobetaine-16 (SB-16), Triton X-200, 3-[(3-cholamidopropyl)dimethylammonium]-1-propanesulfonate (CHAPS), sodium lauroyl sarcosinate (sarcosyl), n-dodecyl-β-D-maltoside (DDM), digitonin, polysorbate 20, and polysorbate 80.

In yet another preferential embodiment of the processes of the present invention, hydrolysis in an acidic environment is carried out with an acid selected from the group consisting of acetic acid, hydrochloric acid, citric acid, lactic acid, and trifluoroacetic acid, preferably acetic acid or hydrochloric acid, more preferably acetic acid.

In yet another preferred embodiment of the processes of this invention, neutralization is by dialysis or performed with a basic solution, preferably a solution comprising sodium hydroxide and/or sodium bicarbonate, optionally in conjunction with a buffer solution comprising one or more of sodium bicarbonate buffer, phosphate-saline buffer, Dulbecco's phosphate-saline buffer, HEPES (4-(2-hydroxyethyl)-1-piperazineethanesulfonic acid), MES (2-(N-morpholino)ethanesulfonic acid), and MOPS (3-(N-morpholino)propanesulfonic acid).

In yet another preferential realization of the processes of the present invention, the tissue or organ of origin is selected from the group consisting of adipose tissue, bone tissue, brain, cartilage tissue, colon, kidney, liver, lung, muscle tissue, myocardial tissue, pancreas, skin, spleen, stomach, vascular tissue, dental tissue, glandular tissue, such as the adrenal gland, the parathyroid gland, the thyroid gland, the pituitary gland, and the salivary gland, bone marrow tissue, tissue of the male reproductive system, such as the testicle, epididymis, prostate, seminal vesicle, and vas deferens, tissue of the female reproductive system, such as the vagina, cervix, endometrium, fallopian tube, ovary, and placenta, intestine, urinary bladder, and eye.

A third object of the present invention refers to a tissue-specific hydrogel, produced by the enzyme-free processes defined in this document. In the case of the process, the first object of the present invention, in particular, the hydrogel is obtained after cross-linking.

A fourth object of the present invention refers to a process for producing a product derived from the tissue-specific extracellular matrix solution, comprising subjecting the tissue-specific extracellular matrix solution produced by the enzyme-free process defined in this document, to one or more of the following chemical modifications:
(i) introduce acrylate derivatives (such as methacrylation);
(ii) introduce alkenes, for thiol-ene chemistry (such as thiol-norbornene);
(iii) introduce thiols, for thiol-ene chemistry or intermolecular disulfide bridges; and/or
(iv) introduce other chemical groups for bioorthogonal reactions (such as aldehyde and hydrazide).

In a preferred realization of the process of the present invention, the process additionally comprises cross-linking the modified tissue-specific extracellular matrix solution for transition to a hydrogel.

A fifth object of the present invention refers to a product derived from the tissue-specific extracellular matrix solution, wherein the product is the tissue-specific solution produced by the enzyme-free process defined in this document, or the tissue-specific hydrogel obtained from it, as defined in this document, or produced by the enzyme-free processes defined in this document, comprising one or more of the following chemical modifications:
(i) addition of acrylate derivatives (such as methacrylation);
(ii) addition of alkenes to thiol-ene chemistry (such as thiol-norbornene);
(iii) addition of thiols for thiol-ene chemistry or intermolecular disulfide bridges; and/or
(iv) addition of other chemical groups for bioorthogonal reactions (such as aldehyde and hydrazide).

A sixth object of the present invention refers to a process for producing a tissue-specific soluble powder, comprising subjecting the tissue-specific neutral extracellular matrix solution, produced by the enzyme-free process defined in this document, or the hydrogel defined in this document, or produced by the enzyme-free processes defined in this document, or the product derived from the extracellular matrix solution defined in this document, or produced by the process to produce a product derived from the extracellular matrix solution defined in this document, to freeze-drying and spraying.

A seventh object of the present invention refers to a tissue-specific soluble powder, obtained by freeze-drying and spraying the tissue-specific neutral extracellular matrix solution, produced by the enzyme-free process defined in this document, or the tissue-specific hydrogel defined in this document, or produced by the enzyme-free processes defined in this document, or the product derived from the extracellular matrix solution defined in this document, or produced by the process to produce a product derived from the extracellular matrix solution defined in this document.

An eighth object of the present invention refers to a process for producing a tissue-specific membrane, comprising subjecting the tissue-specific neutral extracellular matrix solution, produced by the enzyme-free process defined in this document, or the hydrogel defined in this document, or produced by the enzyme-free processes defined in this document, or the product derived from the extracellular matrix solution defined in this document, or produced by the process to produce a product derived from the extracellular matrix solution defined in this document, to dehydration.

A ninth object of this invention refers to a tissue-specific membrane obtained by dehydration of the tissue-specific neutral extracellular matrix solution produced by the enzyme-free process defined in this document, or of the hydrogel defined in this document, or produced by the enzyme-free processes defined in this document, or the product derived from the extracellular matrix solution defined in this document, or produced by the process to produce a product derived from the extracellular matrix solution defined in this document.

A tenth object of the present invention refers to a process for providing bioactive compounds to regenerate tissue using the tissue-specific neutral extracellular matrix solution, produced by the enzyme-free process to produce said solution defined in this document, the hydrogel defined in this document, or produced by the enzyme-free processes defined in this document, or the product derived from the extracellular matrix solution defined in this document, or produced by the process to produce a product derived from the extracellular matrix solution defined in this document, comprising:
(a) load the solution, tissue-specific hydrogel or the product derived from the tissue-specific extracellular matrix solution with the bioactive compound(s); and
(b) apply the solution, the hydrogel or the product derived from the extracellular matrix solution loaded with the bioactive compound(s) on or within the target tissue to be regenerated.

An eleventh object of the present invention refers to a process for providing bioactive compounds to regenerate tissue using the tissue-specific membrane defined in this document, or produced by the process to produce a tissue-specific membrane as defined in this document, comprising:
(a) load the tissue-specific neutral extracellular matrix solution, the tissue-specific hydrogel, or the tissue-specific extracellular matrix solution derivative product with the bioactive compound(s);
(b) dehydrate the solution, the hydrogel or the product derived from the extracellular matrix solution loaded with the bioactive compound(s) to produce a membrane; and
(c) apply the membrane loaded with the bioactive compound(s) on the target tissue to be regenerated;
wherein steps (a) and (b) can occur in any order.

A twelfth object of this invention refers to a process *in vitro* to provide bioactive compounds by means of the tissue-specific hydrogel defined in this document, or produced by the enzyme-free processes defined in this document, in the product derived from the extracellular matrix solution defined in this document, or produced by the process to produce a product derived from the extracellular matrix solution defined in this document, wherein the hydrogel or the product derived from the extracellular matrix solution is for tissue regeneration, comprising loading the tissue-specific hydrogel or the product derived from the tissue-specific extracellular matrix solution with the bioactive compound(s).

In a preferential embodiment of the process of the present invention, the process further comprises dehydrating the hydrogel or the product derived from the extracellular matrix solution loaded with the bioactive compound(s) to produce a membrane.

In another preferred embodiment of this, and other processes of the present invention, the bioactive compound(s) is (are) selected from the group consisting of:
- cells, preferably differentiated adult cells and stem cells; preferably wherein differentiated adult cells are cells derived primarily from endoderm, cells derived primarily from ectoderm, and cells derived primarily from mesoderm; and preferably wherein stem cells are embryonic stem cells, induced pluripotent stem cells, and adult stem cells, or any combination thereof;
- Cell-secreted factors, preferentially selected from the group consisting of growth factors, cytokines, chemokines, and micro-RNAs, or any combination thereof;
- cell-secreted vesicles, preferably selected from the group consisting of exosomes, microvesicles, apoptotic bodies, and ectosomes, or any combination thereof;
- nucleic acids, preferentially selected from the group consisting of deoxyribonucleic acid (DNA), ribonucleic acid (RNA), messenger RNA (mRNA), transfer RNA (tRNA), ribosomal RNAs (rRNA), microRNAs (miRNA), small interfering RNAs (siRNA), piwi-interacting RNAs (piRNA), small nucleolar RNAs (snoRNAs), small nuclear RNAs (snRNA), extracellular RNAs (exRNA), Cajal body-specific small RNAs (scaRNA), long non-coding RNAs (IncRNA), doublestranded RNAs (dsRNA), and circular RNAs, or any combination thereof;
- medications;
- biologic medical products, preferably selected from the group consisting of chimeric antigen receptor T cells (CAR T cells), monoclonal antibodies, fusion proteins, tumor necrosis factor α (TNFα), leukocyte surface glycoproteins, adhesion molecules, immunoglobulins, receptor targets, and viruses, or any combination thereof;
- metallic particles (such as silver nanoparticles), ceramic particles (such as hydroxyapatite), organic particles (such as nanoemulsions and polymeric nanoparticles), lipid particles (such as liposomes) and carbon nanomaterials (such as fullerenes, carbon nanotubes, graphene and graphene oxide), or any combination thereof;
or any combination thereof.

A thirteenth object of this invention refers to the use of the tissue-specific neutral extracellular matrix solution, produced by the process defined in this document, or of the tissue-specific hydrogel defined in this document, or produced by the enzyme-free processes defined in this document, or of the product derived from the extracellular matrix solution defined in this document, or produced by the process to produce a product derived from the extracellular matrix solution defined in this document, for the manufacture of a product.

In a preferential embodiment of the use of the present invention, the product is for tissue regeneration, and wherein the tissue-specific neutral extracellular matrix solution, the tissue-specific hydrogel or the product derived from the tissue-specific extracellular matrix solution is loaded with one or more bioactive compounds, wherein the one or more bioactive compounds are selected from the group consisting of cells, cell-secreted factors, cell-secreted vesicles, nucleic acids, drugs, biological medical products, metallic particles (such as silver nanoparticles), ceramic particles (such as hydroxyapatite), organic particles (such as nanoemulsions and polymeric nanoparticles), lipid particles (such as liposomes) and carbon nanomaterials (such as fullerenes, carbon nanotubes, graphene, and graphene oxide), or a combination thereof.

In another preferred realization of the use of the present invention, the product is selected from the group consisting of a tissue-specific soluble powder, a tissue-specific membrane, a loading carrier for bioactive compounds, such as for regenerating tissue, an additive ingredient for cell culture medium, a bioink for 3D bioprinting, and a surface coating solution for cell culture.

A fourteenth object of this invention refers to the use of the tissue-specific neutral extracellular matrix solution produced by the process defined in this document, or of the hydrogel defined in this document, or produced by the enzyme-free processes defined in this document, or of the product derived from the tissue-specific extracellular matrix solution defined in this document, or produced by the process to produce a product derived from the tissue-specific extracellular matrix solution defined in this document, as a tissue-specific soluble powder, as a tissue-specific membrane, as a loading carrier of bioactive compounds, such as to regenerate tissue and grow cells and/or cell aggregates, as an additive ingredient to cell culture medium, as a bioink for 3D bioprinting, and as a surface coating solution for cell culture.

A fifteenth object of the present invention refers to the use of tissue-specific extracellular matrix solution, produced by the process defined in this document, for the manufacture of derived products, wherein the products comprise the solution with one or more of the following chemical modifications:
(i) addition of acrylate derivatives (such as methacrylation);
(ii) addition of alkenes to thiol-ene chemistry (such as thiol-norbornene);
(iii) addition of thiols to thiol-ene chemistry or intermolecular disulfide bridges; and/or
(iv) addition of other chemical groups for bioorthogonal reactions (such as aldehyde and hydrazide).

A sixteenth object of the present invention refers to an additive ingredient for cell culture medium, comprising the tissue-specific soluble powder defined in this document.

A seventeenth object of the present invention refers to a process for cultivating cells within the hydrogel defined in this document, or produced by the enzyme-free processes defined in this document, or from the product derived from the extracellular matrix solution defined in this document, or produced by the process to produce a product derived from the extracellular matrix solution defined in this document, wherein the process comprises:
(a) load the tissue-specific neutral extracellular matrix solution, produced by the enzyme-free process defined in this document or the derived product of the tissue-specific extracellular matrix solution with the cells, at a cell concentration between 1 × 10³ and 1 × 10¹⁰ cells/mL;
(b) cross-linking the tissue-specific neutral extracellular matrix solution or the derived product of the tissue-specific extracellular matrix solution with the cells to produce a cross-linked 3D construct; and
(c) provide cells with growth conditions in such a way as to allow them to populate the 3D lattice construct to form mature functional tissue.

An eighteenth object of this invention refers to a process for cultivating cellular aggregates within the hydrogel defined in this document, or produced by the enzyme-free processes defined in this document, or from the product derived from the extracellular matrix solution defined in this document, or produced by the process to produce a product derived from the extracellular matrix solution defined in this document, wherein the process comprises:
(a) load the tissue-specific neutral extracellular matrix solution produced by the enzyme-free process defined in this document or the product derived from the tissue-specific extracellular matrix solution with cell aggregates such as spheroids, organoids, and cell monolayers;
(b) cross-linking the tissue-specific neutral extracellular matrix solution or the derived product of the tissue-specific extracellular matrix solution with the cell aggregates to produce a cross-linked 3D construct; and
(c) provide cell aggregates with conditions for growth or maintenance.

A nineteenth object of this invention refers to a process for bioprinting 3D bioinks, comprising cells or cell aggregates within the tissue-specific hydrogel defined in this document, or produced by the enzyme-free processes defined in this document, or from the product derived from the extracellular matrix solution defined in this document, or produced by the process to produce a product derived from the extracellular matrix solution defined in this document, or the tissue-specific extracellular matrix solution, produced by the enzyme-free process to produce a tissue-specific extracellular matrix solution defined in this document, wherein the process comprises:
(a) load cells or cell aggregates into the tissue-specific hydrogel or the derived product of the tissue-specific extracellular matrix solution or the tissue-specific neutral extracellular matrix solution, or in combinations of such hydrogels or products or solutions with other biomaterials;
(b) load the mixture described in item (a) into the printing chamber of a 3D bioprinter;
(c) perform the 3D printing steps, according to the mechanism of action of the 3D bioprinter;
(d) reticular to stabilize the bioprinted 3D construction; and
(e) provide cells with growth conditions in such a way as to allow them to populate the bioprinted 3D construct to form a mature functional tissue.

One twentieth object of the present invention refers to a process for 3D bioprinting, from a tissue-specific extracellular matrix solution, comprising:
produce a tissue-specific extracellular matrix solution by means of steps (a) and (b) of the enzyme-free process to produce such solution as defined in this document;
(c) load the hydrolyzed extracellular matrix solution from step (b) into the printing chamber of a 3D bioprinter;
(d) perform the 3D printing steps, according to the mechanism of action of the 3D bioprinter; and
(e) reticulate to stabilize the bioprinted 3D construction.

In a preferential realization of the 3D bioprinting process of the present invention, between steps (b) and (c), the step of neutralizing the pH of the hydrolyzed extracellular matrix solution from step (b) is carried out to obtain a tissue-specific neutral extracellular matrix solution.

In another preferred embodiment of the process for 3D bioprinting of the present invention, the pH neutralization step of the hydrolyzed extracellular matrix solution is carried out during or after the bioprinting of step (d) or after crosslinking to stabilize the bioprinted 3D construction of step (e).

In yet another preferential realization of the processes for 3D bioprinting of the present invention, between steps (b) and (c), the step of incorporation of biomaterials and/or bioactive compounds is carried out.

In yet another preferred embodiment of the processes for 3D bioprinting of the present invention, the 3D bioprinter printing chamber comprises a syringe, a Petri dish, a flask, or another 3D bioprinting chamber.

In yet another preferred embodiment of the processes for 3D bioprinting of the present invention, the 3D bioprinter is an extrusion-based bioprinter, a stereolithography-based bioprinter, an inkjet-based bioprinter, a laser-based bioprinter, a tomography-based bioprinter, an acoustics-based bioprinter or an electrospinning-based bioprinter.

In yet another preferential embodiment of the processes for 3D bioprinting of the present invention, as well as other processes of the present invention, where relevant, crosslinking preferably comprises one or more of the following: physical crosslinking, such as by changing temperature and/or pH, and adding ions to promote ionic interaction; chemical crosslinking, such as reactions to obtain covalent bonds, such as by exposure to light, including visible light, ultraviolet light, and infrared light.

A twenty-first object of the present invention refers to a kit, comprising the tissue-specific extracellular matrix solution produced by the enzyme-free process to produce said solution defined in this document, or the tissue-specific soluble powder defined in this document, or produced by the process to produce said powder defined in this document, or a product derived from the tissue-specific extracellular matrix solution defined in this document, or produced by the process to produce said product defined in this document.

It is worth noting that any of the objects or their preferred realizations described above can serve as a basis for composing the other objects and their preferred realizations, even if such relationship(s) has not been explicitly described.

### Detailed Description of the Invention

Throughout this document, unless otherwise indicated, the limits of a range of values are included in that range. Also, the value ranges include all the numbers and fractions encompassed within the respective ranges.

Throughout this document, the singular forms "a", "an", and "the" include both the singular and the corresponding plurals, and vice versa, unless the context clearly dictates otherwise.

Throughout this document, the expression "at least one" is equivalent to the expression "one or more", and means one or more members, or at least one member of a group of members. These terms include any of ≥ 1, ≥ 2, ≥ 3, ≥ 4, ≥ 5, ≥ 6, ≥ 7, etc. of said members, and even all said members.

Throughout this document, the terms "about" or "approximately", when referring to a measurable value such as a parameter, a quantity, a temporary duration, and the like, mean variations from the specified value, such as variations of +/-10% or less, preferably +/-5% or less, more preferably +/-1% or less, and even more preferably +/-0.1% or less, to the extent that such variations are reasonable for each applicable parameter of the invention. It is understood that the value to which the modifier "about" refers is also specifically, and preferably, revealed.

Throughout this document, words and expressions such as "preferably", "particularly", "for example", "as", "such as", "more particularly", "more preferably" and the like, and their variations, should be interpreted as entirely optional characteristics, preferred embodiments, or non-exhaustive possible examples, without conferring a limiting character in scope.

Throughout this document, the word "comprises", and any variations such as "comprise" or "comprising", should be interpreted as "open terms", and may imply the inclusion of additional elements or groups of elements, which have not been explicitly described and are not limiting. Likewise the words "including" and "encompassing" and any variations.

Throughout this document, the word "consists", and any variations such as "consist" or "consisting", should be interpreted as "closed terms", and cannot imply the inclusion of additional elements or groups of elements, which were not explicitly described, having a restrictive character.

Where not explicitly stated otherwise, all acronyms, expressions, and/or technical terms shall be construed as having the meanings generally used and widely known in the technical field of this invention. In some cases, terms with commonly understood meanings are defined in this document for the purpose of clarity and/or for ready reference, and the inclusion of such definitions in this document should not necessarily be construed as representing a substantial difference from what is generally understood in the state of the art.

Unless explicitly stated otherwise, the techniques and procedures described in or referred to in this document are generally well understood and employed using conventional methodology, based on the available literature and the knowledge of a person skilled in the art.

Throughout this document, all headings and subheadings are used for convenience only and should not be construed as limitations of this invention.

Throughout this document, all cited references, books, articles, patent documents, and others shall be construed as incorporated by reference.

Throughout this document, the term "tissue-specific" should be interpreted as including the term "organ-specific", and vice versa, so that it encompasses any and all tissues or organs, regardless of the term used.

According to the present invention, preferred tissues or organs of origin for the production of tissue- or organ-specific hydrogels belong to any pertinent animal species or human, and are selected from the group consisting of adipose tissue, bone tissue, brain, cartilage tissue, colon, kidney, liver, lung, muscle tissue, myocardial tissue, pancreas, skin, spleen, stomach, vascular tissue, dental tissue, glandular tissue, such as adrenal gland, parathyroid gland, thyroid gland, pituitary gland and salivary gland, bone marrow tissue, male reproductive system tissue, such as testicle, epididymis, prostate, seminal vesicle and vas deferens, female reproductive system tissue, such as the vagina, cervix, endometrium, fallopian tube, ovary and placenta, intestine, urinary bladder and eye.

Throughout this document, the term "bioactive compound" is broad and should be interpreted as encompassing any compound, component, or active ingredient that exerts an effect on a living organism, tissue, organ, or cell. Preferably, the bioactive compound (or component or active ingredient) is selected from the group consisting of cells (such as stem cells, such as embryonic stem cells, induced pluripotent stem cells, adult stem cells, cells derived primarily from endoderm, cells derived primarily from ectoderm, and cells derived primarily from the mesoderm, or a combination thereof); factors secreted by cells (such as growth factors, cytokines, chemokines, and micro-RNAs, or a combination thereof); vesicles secreted by cells (such as exosomes, microvesicles, apoptotic bodies, and ectosomes, or a combination thereof); nucleic acids (deoxyribonucleic acid (DNA), ribonucleic acid (RNA), messenger RNA (mRNA), transfer RNA (tRNA), ribosomal RNAs (rRNA), microRNAs (miRNA), small interference RNAs (siRNA), piwi interaction RNAs (piRNA), small nucleolar RNAs (snoRNAs), small nuclear RNAs (snRNA), extracellular RNAs (exRNA), Cajal body-specific small RNAs (scaRNA), long non-coding RNAs (IncRNA), doublestranded RNAs (dsRNA), and circular RNAs, or a combination thereof); medicines; biological medical products (such as chimeric antigen receptor T cells (CAR T cells), monoclonal antibodies, fusion proteins, tumor necrosis factor α (TNFα), leukocyte surface glycoproteins, adhesion molecules, immunoglobulins, receptor targets, and viruses, or a combination thereof); metallic particles (such as silver nanoparticles); ceramic particles (such as hydroxyapatite); organic particles (such as nanoemulsions and polymeric nanoparticles); lipid particles (such as liposomes); and carbon nanomaterials (such as fullerenes, carbon nanotubes, graphene, and graphene oxide); or a combination thereof.

Throughout this document, the expression "regenerate tissue" refers to both the regeneration, which can be total or partial (repair), of tissues and organs, and the terms "regenerate," "regeneration," and their variations, in this context, refer to the ability to bring an injured or imperfect tissue to normal or improved conditions, *in vivo* or *in vitro.*

Throughout this document, the term "crosslinking", "reticular" and any variations thereof shall be interpreted as comprising any known crosslinking techniques in the art field of this invention, preferably comprising one or more of the following: physical crosslinking, such as by change in temperature (including increase or decrease in temperature, including freezing) and/or pH, and addition of ions to promote ionic interaction; and chemical crosslinking, such as reaction to obtain covalent bonds, such as by exposure to light, such as visible light, ultraviolet light, and infrared light. Preferably, cross-linking is by temperature change, and can be performed by several known techniques, such as through the use of an incubator. Parameters such as exposure time to a given temperature may vary and be adjusted by a person skilled in the art, without incurring in undue experimentation, depending on the tissue in question. Usually, the temperature for transition from the extracellular matrix solution to a hydrogel is about 4 °C to about 50 °C. Preferably, the temperature is about 15 °C to about 45 °C; more preferably it is about 20 °C to about 40 °C; even more preferably it is about 25 °C to about 38.5 °C; even more preferably it is about 37 °C. Usually, using an incubator with temperature control, relative humidity of 95%, and a CO₂ level of 5%, the time is about 15 minutes to about 2 hours, preferably about 1 hour, at a temperature of 37 °C.

The present invention refers to an enzyme-free process to produce a tissue-specific extracellular matrix solution based on decellularized extracellular matrix, comprising:
(a) decellularize a tissue or organ to obtain the isolated extracellular matrix; and
(b) hydrolyze the isolated extracellular matrix in an acidic environment, in the absence of proteolytic enzymes, optionally followed by purification of the solution.

In a preferential process realization of the present invention, after step (b), proceed with step (c) of neutralizing the pH of the hydrolyzed extracellular matrix solution from step (b) to obtain a tissue-specific neutral extracellular matrix solution.

It should be noted that, optionally, after hydrolysis, and before neutralization, when present, it may be of interest to proceed with the purification of the solution. Purification, even when not specifically indicated, may be present in any of the processes revealed by this document which include these steps, and may be accomplished by any technique known in the field art of the present invention for the removal of impurities. Preferred techniques comprise centrifugation and/or filtration. Centrifugation can preferably occur from about 1,000 g to about 100,000 g, preferably from about 15,000 g to about 90,000 g, for about 1 minute to about 1 hour, preferably for about at least 15 minutes to about 45 minutes; and filtration through membranes, such as membranes with pores ranging preferably between 0.22 µm and 70 µm, more preferably between 1 µm and 10 µm.

To obtain a tissue-specific hydrogel according to the process of the present invention, after step (c), step (d) involves crosslinking the tissue-specific neutral extracellular matrix solution to transition to a hydrogel.

The present invention also refers to an enzyme-free process to produce a tissue-specific hydrogel, based on decellularized extracellular matrix, comprising
produce a tissue-specific extracellular matrix solution by means of steps (a) and (b) of the enzyme-free process of the present invention to produce said solution;
(c) neutralize the pH of the hydrolyzed tissue-specific extracellular matrix solution from step (b) to obtain a neutral tissue-specific extracellular matrix solution; and
(d) cross-link the tissue-specific neutral extracellular matrix solution for transition to a hydrogel.

Similarly, optionally, after hydrolysis and prior to neutralization, it may be of interest to proceed with the purification of the solution as previously described herein.

It may be advantageous, as an optional step of the enzyme-free process to produce the tissue-specific extracellular matrix solution, as well as to produce the tissue-specific hydrogels of the present invention, between steps (a) and (b), to proceed with the step of lyophilizing the isolated extracellular matrix and pulverizing the lyophilized material to obtain a fine powder of isolated extracellular matrix, which makes the hydrolysis of step (b), as well as the process as a whole, faster.

Optionally, in the case of the enzyme-free processes of this invention for the production of a tissue-specific hydrogel, the freeze-drying step of the hydrolyzed extracellular matrix solution is carried out between steps (c) and (d), followed by the resolubilization steps with an aqueous solution, the pH may or may not be adjusted, preferably to about 7 to about 7.4; preferably, the resolubilization is with an acid solution, followed by neutralization to the said preferred pH, and balance of salts.

According to the present invention, any technique of decellularization of a tissue or organ of the state of the art to obtain the isolated extracellular matrix can be employed. There are many decellularization processes described in the state of the art, such as mechanical disruption, freeze-thaw cycles, treatment with osmotic, hyposmotic, and hyperosmotic solutions, treatment with enzymes, treatment with alcohols, acids, bases, and detergents, among others. Preferably, decellularization of the tissue or organ to obtain the isolated extracellular matrix is performed by detergent treatments, such as with Triton X-100, sodium dodecyl sulfate (SDS), sodium lauryl ether sulfate (SLES), sodium cholate, sodium deoxycholate, sulfobetaine-10 (SB-10), sulfobetaine-16 (SB-16), Triton X-200, 3-[(3-cholamidopropyl)dimethylammonium]-1-propanesulfonate (CHAPS), sodium lauroyl sarcosinate (sarcosyl), n-dodecyl-β-D-maltoside (DDM), digitonin, polysorbate 20, and polysorbate 80. Preferred detergents according to the present invention are Triton X-100, sodium dodecyl sulfate (SDS), sodium lauryl ether sulfate (SLES), sodium deoxycholate, Triton X-200 and 3-[(3-colamidopropyl)dimethylammonium]-1-propanesulfonate (CHAPS). Even more preferentially, the detergents are Triton X-100, sodium dodecyl sulfate (SDS), and sodium lauryl ether sulfate (SLES).

Preferably, the decellularization protocol includes dissection of native tissue; freeze/thaw cycles of native tissue; milling of native tissue; followed by water washes; incubation with deionized water; incubation with water saturated in sodium chloride; incubation with sodium lauryl ether sulfate (SLES) or sodium dodecyl sulfate (SDS) of 0.1% to 2% for 12 to 48 h, preferably at 1% for 24 hours; water washes; incubation with 0.1% to 2% Triton X-100 for 12 to 48 h, preferably 1% for 24 hours; and water washes. The technician in the subject will be able to readily recognize variations of the described protocol to properly obtain the decellularization of the target tissue by the method described, the number of washes, freezing and thawing cycles, among other steps, not being of particular relevance to the present invention.

Likewise, in any case according to the present invention, any freeze-drying technique described in the state of the art may be employed. Usual techniques generally used for freeze-drying employ a freeze dryer, according to the standard methodology of each equipment. Any equipment may be used in the context of the present invention.

Likewise, any technique of spraying the lyophilized material to obtain a fine powder, described in the state of the art, can be employed. Usual techniques for pulverization include equipment for this purpose, such as a mill (impact mill, cutting mill (knife mill), among others). Preferably, freeze-drying is from -65 °C to -35 °C for 12 to 48 hours, preferably at -45 °C for 24 hours, followed by pulverization in a Willey-type knife mill.

The hydrolysis of the isolated extracellular matrix, whether or not it has been lyophilized and pulverized (therefore, whether or not it is in the form of a fine extracellular matrix powder), according to the present invention, should be performed in an acidic environment and in the absence of any proteolytic enzymes, usually at 1 to 100 mg/mL of lyophilized extracellular matrix powder, preferably 5 to 20 mg/mL and even more preferably at 10 mg/mL of lyophilized extracellular matrix powder. Usual acid hydrolysis techniques include the use of an organic or inorganic acid, which can be a weak acid or a strong acid, such as acetic acid, hydrochloric acid, citric acid, lactic acid, and trifluoroacetic acid. The acid concentration can vary depending on the acid in question, ranging from 0.001 M to 2 M. Preferably, hydrolysis in an acidic environment is performed with acetic acid or hydrochloric acid, preferably acetic acid, preferably acetic acid at 0.5 M. The hydrolysis time can vary depending on the tissue in question, and can range from a few hours to a few days, or even weeks. It may be advantageous to keep the hydrolysis under stirring at a cooled temperature, such as in a refrigerator at about 4 °C. A person skilled in the art would know how to implement small improvements in this stage and its variables, depending on the acid and fabric in question.

Neutralization of the pH of the hydrolyzed extracellular matrix solution from the previous step to obtain a tissue-specific neutral extracellular matrix solution can be performed by any technique usually employed for this purpose. Known techniques include the use of a basic solution, preferably a solution comprising sodium hydroxide and/or sodium bicarbonate. Optionally, when salt balancing is required, a buffer solution comprising one or more of the sodium bicarbonate buffer, phosphate-saline buffer, Dulbecco's phosphate-saline buffer, HEPES (4-(2-hydroxyethyl)-1-piperazineethanesulfonic acid), MES (2-(N-morpholino)ethanesulfonic acid), and MOPS (3-(N-morpholino)propanesulfonic acid) may be used in conjunction with the basic solution, by mixing the basic solution with the buffer solution, and with the hydrolyzed extracellular matrix. In general, the basic solution, with or without buffer(s), acts immediately to neutralize the acid, and there can also be incubation for a few minutes to about a few hours. The quantities of the reagents and other conditions can be adjusted depending, for example, on the acid in question, using standardization techniques that are usual for a person skilled in the art. An example of a preferred solution for neutralizing the pH of hydrolyzed extracellular matrix solution (neutralization buffer) is: 16% w/v sodium hydroxide (NaOH), 3.7% w/v sodium bicarbonate (NaHCO₃) and 4.8% w/v HEPES, being mixed into the acid matrix, for example, in a ratio of 8 (acid matrix): 1 (neutralization buffer) : 1 (DMEM (Dulbecco's Modified Eagle's Medium) 10X). Alternatively, pH neutralization of the hydrolyzed extracellular matrix solution can be accomplished by dialysis. It is a methodology known in this technical field for this purpose, and adjustments can be made by a technician in the subject without undue experimentation. Briefly, the hydrolyzed solution is placed in a dialysis bag with adequate pores sufficient to allow the passage of salts and water, but not proteins. By changing the water bath wherein this bag is inserted, the pH is adjusted to the desired neutral point. Preferably, according to this step of the present invention, neutralization is performed with a sodium hydroxide solution (with buffer) or dialyzed. In the context of the present invention, preferably, and regardless of the technique, the pH of the neutralized solution is 6.8 to 8.0, preferably 7.0 to 7.4.

Still in the case of the processes to produce a tissue-specific hydrogel of the present invention, when applicable, after proceeding with the lyophilization of the hydrolyzed solution of the extracellular matrix, by the techniques known and already informed of the state of the art, the resolubilization step follows with an aqueous solution, and the pH may or may not be adjusted, preferably to about 7 to about 7.4; preferably, resolubilization is with an acid solution, such as with the acids mentioned above in the context of acid hydrolysis, preferably with 0.5 M acetic acid. After complete solubilization, it is followed, in the case of the use of acid, with the neutralization to the said preferential pH, and equilibrium of the salts of said solution, as well as by the neutralization and salt equilibrium methods described above, preferably with the neutralization buffer solution reported above (16% w/v NaOH, 3.7% w/v NaHCO₃; 4.8% w/v HEPES), in the reported ratio of solubilized matrix, neutralization buffer, and 10x concentrated culture medium in the ratio of 8:1:1. Advantageously, this allows for obtaining a final powder product with a longer shelf life, as well as enabling work with solutions of varying concentrations from resolubilization in mass/volume ratios of interest.

It is worth noting that, when it is chosen, between steps (c) and (d), to lyophilize the hydrolyzed solution of extracellular matrix, resolubilize with an acid solution, neutralize and balance the salts, the neutralization of step (c) is preferably performed with a basic solution, preferably a solution comprising sodium hydroxide and/or sodium bicarbonate, without buffer.

It is also worth noting that, depending on the use of interest, in an entirely optional way, one can proceed with the sterilization of the components and/or the extracellular matrix and/or hydrogel of the present invention. This can be carried out in various ways and at different times, being within the knowledge of a person skilled in the art of the present invention.

The present invention further refers to a process for producing a product derived from the tissue-specific extracellular matrix solution which comprises subjecting the tissue-specific extracellular matrix solution, produced by the enzyme-free process of the present invention, to one or more of the following chemical modifications:
(i) introduce acrylate derivatives (such as methacrylation);
(ii) introduce alkenes, for thiol-ene chemistry (such as thiol-norbornene);
(iii) introduce thiols, for thiol-ene chemistry or intermolecular disulfide bridges; and/or
(iv) introduce other chemical groups for bioorthogonal reactions (such as aldehyde and hydrazide).

Preferably, the process additionally comprises cross-linking the tissue-specific extracellular matrix solution modified for transition to a hydrogel.

Optionally, one can further proceed from said modified extracellular matrix solution or the hydrogel derived from it, with the following steps: freeze-drying and spraying of said material to obtain a fine powder. Optionally, resolubilization with an aqueous solution can then be carried out, and the pH may or may not be adjusted, preferably to about 7 to about 7.4; preferably, resolubilization is with an acid solution, followed by neutralization to the said preferred pH, and salt balance. The protocols and reagents in question are as described herein for equivalent steps of other processes and/or will depend on the reactions of particular chemical modifications, as known by a person skilled in the art.

All reactions are known from the literature and can be used as a basis for the realization of this invention, without undue experimentation. In particular, acrylate derivatives can be introduced into the hydrogel or solution by means of a number of chemical reactions known to a subject matter technician, such as a methacrylation reaction. To perform extracellular matrix methacrylation, in a particular example, the methacrylic anhydride reagent is added dropwise to the hydrolyzed extracellular matrix solution at a ratio of 2.5 mL/g of matrix. The reaction occurs under constant agitation for varying time and temperature, which can change the resulting degree of functionalization. In a preferred example, the reaction occurs for about 48 hours from 4 °C to 24 °C, preferably at room temperature (approximately 22°C). At the end, the reaction product is dialyzed against deionized water for 5 days at room temperature and the pH is neutralized using a sodium bicarbonate solution. The material is then frozen, freeze-dried, and pulverized. Alternatively, methacrylation can be performed by reacting the hydrolyzed extracellular matrix with excess glycidyl methacrylate in the presence of trimethylamine and tetrabutylammonium bromide. In one example, the reaction solution consists of 150 mL of a mixture of acetone and water solvents (1:3), wherein 2.2 mL of triethanolamine, 2.2 mL of glycidyl methacrylate, and 2.2 g of tetrabutylammonium are dissolved. The reaction solution is added to the hydrolyzed matrix at a ratio of 150 mL for every 1g of matrix and then remains under constant stirring for 5 days at room temperature. After the end of this period, the methacrylated extracellular matrix is precipitated with excess ethanol or acetone, centrifuged, and then resolubilized in water. The product is then dialyzed against deionized water for 2 days, with daily water changes, and finally lyophilized and pulverized.

Alkene chemical groups can be introduced into the hydrogel or solution by means of various chemical reactions known to a subject matter technician, such as for thiol-ene chemistry (such as thiol-norbornene). In particular, for the chemical modification of the primary amine groups of the matrix with norbornene, the document Mũnoz, Z.; Shih, H.; Lin, C. C.; "Gelatin hydrogels formed by orthogonal thiol-norbornene photochemistry for cell encapsulation", 2014, is incorporated by reference. Briefly, in one embodiment, one can, for example, react the soluble hydrolyzed extracellular matrix with a carboxylic anhydride (such as cis-5-norbornene-endo-2,3-dicarboxylic anhydride, CAS 129-64-6). The reagent is added to the soluble matrix at the rate of 2 g for every 1 g of matrix, the pH is neutralized with sodium hydroxide, and the reaction is conducted for 3 hours at room temperature. At the end of the period, 5x the volume of phosphate-buffered saline (PBS) is added to stop the reaction, and the reaction mixture is centrifuged to remove any undissolved anhydride. At the end, the reaction product is dialyzed against deionized water for 3 days at room temperature and the pH is neutralized using a sodium bicarbonate solution. The material is then frozen, freeze-dried, and pulverized.

Thiol chemical groups can be introduced into the hydrogel or solution through a variety of chemical reactions known to a subject matter technician. For example, for the thiolation reaction, the document by Petrou, C. L. et al. is incorporated by reference; "Clickable decellularized extracellular matrix as a new tool for building hybrid-hydrogels to model chronic fibrotic diseases in vitro", 2020. In one realization, the soluble hydrolyzed extracellular matrix is reacted with 2-iminothiolane hydrochloride (Traut's reagent). Initially, the primary amine groups of the extracellular matrix must be quantified, which can be done by the ninhydrin assay. Then, the matrix is reacted with excess Traut's reagent in the proportion of 75 molar relative to the primary amine concentration determined in the matrix, in the presence of 2 mM ethylenediaminetetraacetic acid (EDTA) for 2 hours at room temperature. After the reaction, the solution is filtered or dialyzed to remove Traut's reagent and finally the material is freeze-dried and pulverized.

Other chemical groups can be introduced into the hydrogel or solution by various chemical reactions known to a person skilled in the art, such as by bioorthogonal reactions (such as for the insertion of aldehyde and hydrazide), as indicated in the document Koivisto, J.T. et al.; "Mechanically Biomimetic Gelatin-Gellan Gum Hydrogels for 3D Culture of Beating Human Cardiomyocytes," 2019, incorporated by reference. Particular examples include the chemical modification of the extracellular matrix with adipic hydrazide or carbohydrazide. For the chemical modification of the matrix with adipic hydrazide, for example, the soluble hydrolyzed extracellular matrix can be reacted with this reagent in the proportion of 0.225 M of hydrazide for each 1 g of matrix. The pH of the reaction mixture is adjusted to neutral using sodium hydroxide, and the reaction occurs for 24 hours in the presence of 0.03 M carbodiimide (EDC) and 0.03 M hydroxybenzotriazole (HOBt). At the end, the reaction product is dialyzed against deionized water for 2 days at room temperature and the pH is neutralized using a sodium bicarbonate solution. The material is then frozen, freeze-dried, and pulverized.

The introduction of these chemical groups into the hydrogel or solution of the present invention is useful because it allows the mechanical properties and degradation rate of the hydrogels obtained after gelation of the chemically modified extracellular matrix to be manipulated and adjusted according to the needs of different applications, as it opens up the possibility of using different strategies to reinforce the structure of the hydrogel or cross-linking techniques. In other words, in addition to thermal crosslinking, which can contribute to the reinforcement of the product, the modifications allow for the manipulation of these mechanical properties and the control of degradability through strong chemical reactions, which can be mediated by light irradiation, for example (in the case of methacrylation) or by combination with other crosslinking agents (as in the case of thiol-ene chemistry). Depending on the degree of crosslinking (e.g., adding more or fewer chemical groups, applying more or less light, etc.), it is advantageously possible to obtain products with different properties of interest.

Thus, the present invention also refers to a product derived from the tissue-specific extracellular matrix solution, wherein the product is the tissue-specific solution, produced by the enzyme-free process of the present invention, or the tissue-specific hydrogel produced by the enzyme-free processes of the present invention, comprising one or more of these chemical modifications.

Further, the present invention refers to a process for producing a tissue-specific soluble powder, as well as the tissue-specific soluble powder produced by the process, which includes submitting the tissue-specific neutral extracellular matrix solution produced by the process disclosed in this document, or the tissue-specific hydrogel disclosed in this document, or produced by the enzyme-free processes disclosed in this document, or the product derived from the tissue-specific extracellular matrix solution disclosed in this document, or produced by the process to produce a product derived from the tissue-specific extracellular matrix solution disclosed in this document, to freeze-drying and spraying. Freeze-drying and spraying, as already mentioned, can be performed by one of the various state-of-the-art methodologies, some of which have already been pointed out herein. Preferably, the tissue-specific soluble powder is obtained by grinding.

The present invention further refers to a process for producing a tissue-specific membrane, as well as to the tissue-specific membrane produced by the process, which comprises submitting the tissue-specific neutral extracellular matrix solution, produced by the enzyme-free process defined in this document, or the tissue-specific hydrogel disclosed by this document or produced by the enzyme-free processes disclosed by this document, or the product derived from the tissue-specific extracellular matrix solution disclosed herein, or produced by the process to produce a product derived from the tissue-specific extracellular matrix solution disclosed herein, to dehydration. The technique of dehydration is known and widely employed in the state of the art for this purpose. Techniques for dehydrating hydrogels commonly employed include liofilização (*freeze-drying*) and "secagem ao ar" (*air-drying*), whose variables, such as time, temperature, etc., can be easily determined by a person skilled in the art of the present invention. Preferably, the freeze-drying dehydration technique comprises freezing the hydrogel (ideally below -40 °C); placing it in the freeze-drying chamber; cooling the freeze-drying chamber below -40 °C; exposing the frozen material to a vacuum; waiting for hours to days (depending on the power of the freeze dryer, sample size, etc.); turning off the cooling and vacuum and removing the lyophilized sample from the chamber. The "air drying" technique, preferably, involves letting it dry in the open air. Variables such as temperature and humidity can be adjusted to better control the process, such as placing the material in a dry incubator at a high temperature (around 40 °C).

The present invention further refers to a process for providing bioactive compounds to regenerate tissue using the tissue-specific neutral extracellular matrix solution, produced by the enzyme-free process to produce said solution disclosed in this document, the tissue-specific hydrogel disclosed in this document, or produced by the enzyme-free processes disclosed in this document, or the product derived from the tissue-specific extracellular matrix solution disclosed in this document, or produced by the process to produce a product derived from the tissue-specific extracellular matrix solution disclosed in this document, comprising (a) loading the solution, hydrogel, or solution-derived product with the bioactive compound(s); and (b) applying the solution, hydrogel or solution-derived product loaded with the bioactive compound(s) on or within the target tissue to be regenerated. In the case of loading the tissue-specific neutral extracellular matrix solution with the bioactive compound(s), it can be applied directly on or within the target tissue to be regenerated so that it crosslinks into the tissue itself. Any state-of-the-art loading techniques may be used and are known to the person skilled in the art, and are not of particular importance to the present invention. For example, preferably, bioactive compounds can be resuspended in the hydrogel resulting in a hydrogel dilution of about 10%.

The present invention further refers to a process for providing bioactive compounds to regenerate tissue using a tissue-specific membrane disclosed in this document, or produced by the process to produce a tissue-specific membrane disclosed in this document, comprising (a) loading the tissue-specific neutral extracellular matrix solution, the tissue-specific hydrogel or the derived product of the tissue-specific extracellular matrix solution with the bioactive compound(s); (b) dehydrating the solution, the hydrogel or the product derived from the extracellular matrix solution loaded with the bioactive compound(s) to produce a membrane; and (c) applying the membrane loaded with the bioactive compound(s) to the target tissue to be regenerated; wherein steps (a) and (b) can occur in any order, i.e., such process can occur either as (a)-(b)-(c) or as (b)-(a)-(c).

In addition, the present invention also refers to a process *in vitro* to provide bioactive compounds by means of the tissue-specific hydrogel revealed by this document, or produced by the enzyme-free processes disclosed in this document, or in the derived product of the tissue-specific extracellular matrix solution revealed by this document or produced by the process to produce a product derived from the tissue-specific extracellular matrix solution revealed by this document, wherein the hydrogel or the solution-derived product is used for tissue regeneration, comprising loading the hydrogel or the solution-derived product with the bioactive compound(s). Preferably, this latter process also involves dehydrating the hydrogel or the solution-derived product loaded with the bioactive compound(s) to produce a membrane. Loading and dehydration techniques have already been addressed and can equally be used to carry out the present process. It is worth noting that the bioactive compound(s) (one or more bioactive compounds) are preferably selected from the group as specified earlier in this document and can be used alone or in any combination, depending on the use of interest.

This invention further refers to the use of the tissue-specific neutral extracellular matrix solution, produced by the enzyme-free process defined in this document, or the tissue-specific hydrogel revealed by this document or produced by the enzyme-free processes disclosed by this document, or the product derived from the tissue-specific extracellular matrix solution revealed by this document or produced by the process to produce a product derived from the solution revealed by this document for the manufacture of a product. Preferably, the product is for tissue regeneration, and wherein the tissue-specific neutral extracellular matrix solution, the hydrogel or the solution-derived product can be loaded with one or more bioactive compounds as described earlier in this document, either alone or in any combination, depending on the intended use, using usual loading techniques already described. The product, according to the present invention, can be a tissue-specific soluble powder, a tissue-specific membrane, a loading carrier for bioactive compounds, such as for regenerating tissue, an additive ingredient for cell culture media, a bioink for 3D bioprinting and a surface coating solution for cell culture. The products are generally produced according to the processes of the present invention.

This invention also refers to the use of the tissue-specific neutral extracellular matrix solution produced by the process defined in this document, or of the tissue-specific hydrogel disclosed by this document or produced by the enzyme-free processes revealed by this document, or of the product derived from the tissue-specific extracellular matrix solution disclosed by this document or produced by the process to produce a product derived from the tissue-specific extracellular matrix solution disclosed by this document as a tissue-specific soluble powder, as a tissue-specific membrane, as a loading carrier for bioactive compounds, such as for regenerating tissue and cultivating cells and/or cell aggregates, as an additive ingredient for cell culture media, as a bioink for 3D bioprinting, and as a surface coating solution for cell culture.

The present invention also refers to the use of the tissue-specific extracellular matrix solution produced by the enzyme-free process disclosed in this document for the manufacture of derived products, wherein the products comprise the solution with one or more of the following chemical modifications:
(i) addition of acrylate derivatives (such as methacrylation);
(ii) addition of alkenes to thiol-ene chemistry (such as thiol-norbornene);
(iii) addition of thiols for thiol-ene chemistry or intermolecular disulfide bridges; and/or
(iv) addition of other chemical groups for bioorthogonal reactions (such as aldehyde and hydrazide). Methods of carrying out such modifications have been exemplified above.

The present invention also refers to an additive ingredient for cell culture medium, comprising the tissue-specific soluble powder revealed by this document. Once the powder is diluted in the cell culture medium, the cells become exposed to tissue-specific proteins during culture.

The present invention further refers to a process for cultivating cells within the tissue-specific hydrogel disclosed in this document or produced by the enzyme-free processes disclosed in this document, or from the product derived from the tissue-specific extracellular matrix solution disclosed in this document or produced by the process to produce a product derived from the tissue-specific extracellular matrix solution disclosed in this document, comprising (a) loading the tissue-specific neutral extracellular matrix solution, produced by the enzyme-free process defined in this document or the product derived from the tissue-specific extracellular matrix solution with the cells, at a concentration of cells between about 1 × 10³ and about 1 × 10¹⁰ cells/mL. Preferably, the concentration of cells is between about 1 × 10⁴ and about 1 × 10⁹ cells/mL, more preferably between about 1 × 10⁵ and about 1 × 10⁸ cells/mL. Another step in the process of the present invention is to (b) cross-link the tissue-specific neutral extracellular matrix solution or the derived product of the tissue-specific extracellular matrix solution with the cells to produce a cross-linked 3D construct. Yet another step in the process of the present invention is (c) to provide the cells with growth conditions in such a way as to allow them to populate the 3D cross-linked construct to form mature functional tissue. Suitable growth conditions include the specific culture conditions of each tissue or organ, and are known from the state of the art and/or can be readily obtained from the existing literature and scientific publications.

Similar reasoning may be applied to another process of the present invention, which refers to a process of cultivating cellular aggregates within the tissue-specific hydrogel disclosed in this document or produced by the enzyme-free processes disclosed in this document, or from the product derived from the tissue-specific extracellular matrix solution disclosed in this document or produced by the process to produce a product derived from the tissue-specific extracellular matrix solution disclosed in this document, comprising (a) loading the tissue-specific neutral extracellular matrix solution, produced by the enzyme-free process defined in this document, or the product derived from the tissue-specific extracellular matrix solution with the cellular aggregates, such as spheroids, organoids, and cellular monolayers. Another step of the process of the present invention comprises (b) cross-linking the tissue-specific neutral extracellular matrix solution or the derived product of the tissue-specific extracellular matrix solution with the cell aggregates to produce a 3D cross-linked construct. Yet another step in the process of the present invention is (c) to provide cell aggregates with conditions for growth or maintenance. Adequate growth conditions include the specific culture conditions of each tissue or organ, as already discussed. It is worth noting that, both in the case of the process for cultivating cells and in the case of the process for cultivating cell aggregates, according to the present invention, one can optionally proceed with the step of incorporating biomaterials and/or bioactive compounds before step (a), or between steps (a) and (b).

The present invention further refers to a process for bioprinting 3D bioinks comprising cells or cell aggregates within the tissue-specific hydrogel revealed by this document or produced by the enzyme-free processes revealed by this document, or from the product derived from the tissue-specific extracellular matrix solution revealed by this document or produced by the process to produce a product derived from the tissue-specific extracellular matrix solution revealed by this document, or of the tissue-specific extracellular matrix solution, produced by the enzyme-free process to produce a tissue-specific extracellular matrix solution revealed herein, comprising (a) loading cells or cell aggregates into the hydrogel, or the tissue-specific extracellular matrix solution derivative, or in tissue-specific neutral extracellular matrix solution, or in combinations of such hydrogels or products or solutions with other biomaterials. In this context, the expression "other biomaterials" refers to the biological materials generally used for 3D bioprinting, such as natural biomaterials and synthetic biomaterials. Among the natural biomaterials, those produced from fibrin, collagen, elastin, gelatin, polypeptides, hyaluronic acid, polysaccharides (including alginate, agarose, chitosan, pectin, and nanocellulose), and modified natural polymers, as well as their mixtures or compositions containing them, are included, but are not limited to. Among the synthetic biomaterials are those produced from polyethylene glycol, polyacrylic acid, polyvinyl alcohol, polyacrylic acid, polymethyl methacrylate, poly(methacrylic acid), polyacrylamide, polyvinylpyrrolidone, polyurethane, poly(hydroxyethyl methacrylate), poly(N-isopropyl acrylamide), and polyamide, their mixtures or compositions containing them. Other steps in the process of this invention include (b) loading the mixture described in item (a) into the printing chamber of a 3D bioprinter and (c) performing the 3D printing steps, according to the mechanism of action of the 3D bioprinter, which, of course, may vary depending on the equipment in question. In this context, it should be pointed out that any 3D bioprinter can serve for the process of the present invention. Yet another step in the process of the present invention comprises (c) reticulating to stabilize the bioprinted 3D construction. Still, the process of the present invention comprises step (e) of providing cells with growth conditions in order to allow them to populate the bioprinted 3D construct to form a mature functional tissue. Such growing conditions may vary in each case and a technician in the subject, based on this document and/or knowledge of the state of the art, would be able to obtain the mentioned stand without improper experimentation.

The present invention also refers to a process for 3D bioprinting from a tissue-specific extracellular matrix solution, comprising producing a tissue-specific extracellular matrix solution through steps (a) and (b) of the enzyme-free process disclosed in the present document; (c) loading the hydrolyzed extracellular matrix solution from step (b) into the printing chamber of a 3D bioprinter; (d) performing the 3D printing steps according to the mechanism of action of the 3D bioprinter; and (e) crosslinking to stabilize the bioprinted 3D construction. Examples of techniques from the respective stages were previously described in this document. Preferably, between steps (b) and (c), the step of neutralizing the pH of the hydrolyzed extracellular matrix solution from step (b) is carried out to obtain a tissue-specific neutral extracellular matrix solution, according to the neutralization techniques mentioned previously. Alternatively, one can proceed with neutralizing the pH of the hydrolyzed extracellular matrix solution during or after the bioprinting of step (d) or after performing the crosslinking step to stabilize the bioprinted 3D construct of step (e). In any case, preferably, between steps (b) and (c), more preferably after neutralizing the pH of the hydrolyzed extracellular matrix solution of step (b), if present, proceed with the step of incorporating biomaterials and/or bioactive compounds.

According to the processes for 3D bioprinting (process for bioprinting 3D bioinks and process for 3D bioprinting, from an enzyme-free tissue-specific extracellular matrix solution), preferably, the 3D bioprinter printing chamber comprises a syringe, a Petri dish, a flask, or another 3D bioprinting chamber. Also, the 3D bioprinter can be of any type, such as an extrusion-based bioprinter, a stereolithography-based bioprinter, an inkjet-based bioprinter, a laser-based bioprinter, a tomography-based bioprinter, an acoustics-based bioprinter, or an electrospinning-based bioprinter. Also, according to the 3D bioprinting processes of the present invention, crosslinking preferably comprises one or more of the following: physical crosslinking, such as by changing temperature and/or pH, and adding ions to promote ionic interaction; and chemical crosslinking, such as reactions to form covalent bonds, such as by exposure to light, including visible light, ultraviolet light, and infrared light.

Finally, the present invention refers to a kit comprising the tissue-specific extracellular matrix solution, produced by the enzyme-free process to produce a tissue-specific extracellular matrix solution revealed by this document, or the tissue-specific soluble powder revealed by this document, or produced by the process to produce such powder, or a product derived from the tissue-specific extracellular matrix solution revealed by this document, or produced by the process to produce a product derived from the tissue-specific extracellular matrix solution revealed by this document. Preferably, the kit comprises a basic solution in conjunction with a buffer solution. Preferably, the basic solution in conjunction with the buffer solution is 16% w/v sodium hydroxide (NaOH), 3.7% w/v sodium bicarbonate (NaHCO₃), 4.8% w/v HEPES, and 10x concentrated culture medium.

The preferred characteristics and achievements of the processes and products of the present invention, as described above, can also be foreseen for the present uses, and vice versa.

The technical characteristics of the processes of this invention can be better understood on the basis of the examples below, and should therefore not be interpreted in a restrictive manner.

### Example 1

### Enzyme-free process to produce tissue-specific hydrogels

Decellularization of porcine heart tissue was initially achieved by dissection to separate the aortic tissue from other cardiac tissues. Afterwards, the native tissue was subjected to a cycle of freezing at -20 °C for 24 hours and thawing at room temperature for 24 hours. Subsequently, the tissue was ground in a semi-industrial grinder until the tissue fragments were smaller than 2 mm, followed by 5 washes with water, and incubation of the resuspended material with deionized water at room temperature for 3 hours. After this period, the supernatant fluid was discarded, followed by tissue resuspension and incubation at room temperature for 3 hours, this time with water saturated with sodium chloride, followed by 3 water washes, and incubation of the material with 1% sodium lauryl ether sulfate (SLES) or sodium dodecyl sulfate (SDS) for 24 hours; afterwards, the tissue was subjected to 5 more water washes and incubation with 1% Triton X-100 for 24 hours, followed by 5 new water washes.

After decellularization, the decellularized extracellular matrix was lyophilized at -45 °C for 24 hours and ground with a Willey-type mill.

Subsequently, the hydrolysis of the isolated extracellular matrix powder at 10 mg/mL in 0.5 M acetic acid was carried out for 1 week at 4 °C under constant agitation, in the absence of proteolytic enzymes. Afterwards, the solution of decellularized extracellular matrix hydrolyzed at 15,000 x g was centrifuged for 10 minutes to remove non-hydrolyzed particles; the supernatant was separated and the sedimented material was discarded.

Subsequent neutralization of the supernatant was performed using a neutralizing buffer solution containing 2.2% sodium bicarbonate and 15% sodium hydroxide in phosphate-saline buffer, followed by incubation of the neutralized solution of decellularized extracellular matrix at 37 °C for 1 hour in an incubator with control of CO₂ and humidity for transition to a hydrogel.

### Example 2

### Enzyme-free process to produce tissue-specific hydrogels

Tissue decellularization was performed according to the methodology described in Example 1. Subsequently, the hydrolysis of the isolated extracellular matrix powder at 10 mg/mL in 0.5 M acetic acid was carried out for 1 week at 4 °C under constant agitation, in the absence of proteolytic enzymes. Afterwards, the neutralization of the pH of the hydrolyzed extracellular matrix solution was achieved with the addition of a basic solution (neutralization buffer) containing 16% w/v NaOH, 3.7% w/v NaHCO₃, 4.8% w/v HEPES, mixed with the acid matrix in a ratio of 8 (acid matrix): 1 (neutralization buffer) : 1 (DMEM 10x Sigma D2429), followed by incubation of the neutralized decellularized extracellular matrix solution at 37 °C for 1 hour in an incubator with CO₂ and humidity control for transition to a hydrogel.

### Example 3

### Process to produce a product derived from a tissue-specific extracellular matrix solution

The extracellular matrix solution hydrolyzed at 10 mg/mL in acetic acid at 0.5 M for 1 week at 4 °C under constant agitation, as described in the previous example, was prepared and at the end of the process its pH was adjusted to 8 using sodium hydroxide (NaOH) solution at 0.1 M. To perform the extracellular matrix methacrylation reaction, the methacrylic anhydride reagent was added to the pH 8 solution, drop by drop, in the proportion of 2.5 mL for every 1 g of extracellular matrix, and then it remained under constant agitation for 48 hours at room temperature (about 22 °C). At the end of the period, the methacrylated matrix obtained was dialyzed against deionized water for 5 days at room temperature, with daily water changes, to remove the unreacted methacrylic acid and anhydride. The pH correction to 7 (neutralization) was performed at the end of dialysis using a 0.1 M sodium bicarbonate (NaHCO₃) solution. The neutralized solution was then frozen, lyophilized, and subsequently pulverized. The material obtained was sterilized by exposure to ethylene oxide. The methacrylate extracellular matrix was then resolubilized in an acid solution (0.5 M acetic acid) at concentrations between 1% and 3%, and after complete solubilization, the solution was neutralized and the salts were balanced using the neutralization buffer solution (16% w/v NaHCO₃; 4.8% p/v HEPES). For this, the solubilized methacrylated extracellular matrix was mixed with the neutralization buffer and 10x concentrated culture medium in the ratio of 8:1:1, respectively. The product derived from the tissue-specific extracellular matrix solution, obtained by the addition of acrylate groups by methacrylation as described above, is photocrosslinkable when combined with a photoinitiator.

## Claims

1. ENZYME-FREE PROCESS TO PRODUCE A TISSUE-SPECIFIC EXTRACELLULAR MATRIX SOLUTION, based on a decellularized extracellular matrix, **characterized in that** comprises:
(a) decellularize a tissue or organ to obtain the isolated extracellular matrix; and
(b) hydrolyze the isolated extracellular matrix in an acidic environment, in the absence of proteolytic enzymes, optionally followed by purification of the solution.

2. PROCESS, according to claim 1, **characterized in that** after step (b), proceeds with step (c) of neutralizing the pH of the hydrolyzed extracellular matrix solution from step (b) to obtain a tissue-specific neutral extracellular matrix solution.

3. PROCESS, according to claim 2, **characterized in that**, after step (c), proceeds with step (d) of cross-linking the tissue-specific neutral extracellular matrix solution for transition to a hydrogel.

4. ENZYME-FREE PROCESS TO PRODUCE A TISSUE-SPECIFIC HYDROGEL, based on a decellularized extracellular matrix, **characterized in that** comprises:
produce a tissue-specific extracellular matrix solution through steps (a) and (b) of the enzyme-free process as defined in claim 1;
(c) neutralize the pH of the hydrolyzed tissue-specific extracellular matrix solution from step (b) to obtain a tissue-specific neutral extracellular matrix solution; and
(d) cross-link the tissue-specific neutral extracellular matrix solution for transition to a hydrogel.

5. PROCESS, according to any of claims 1 to 4, **characterized in that**, between steps (a) and (b), proceeds with the step of lyophilizing the isolated extracellular matrix and spraying the lyophilized material to obtain a fine powder of isolated extracellular matrix.

6. PROCESS, according to any of claims 3 to 5, **characterized by**, between steps (c) and (d), proceeding with the steps of lyophilizing the hydrolyzed solution of extracellular matrix, resolubilizing with an aqueous solution, with or without pH adjustment; preferably, resolubilization is with an acidic solution, followed by neutralization and salt balance.

7. PROCESS, according to any of claims 1 to 6, **characterized in that** decellularization is performed with at least one detergent selected from the group consisting of Triton X-100, sodium dodecyl sulfate (SDS), sodium lauryl ether sulfate (SLES), sodium cholate, sodium deoxycholate, Sulfobetaine-10 (SB-10), Sulfobetaine-16 (SB-16), Triton X-200, 3-[(3-cocamidopropyl)dimethylammonium]-1-propanesulfonate (CHAPS), sodium lauroyl sarcosinate (sarcosyl), n-dodecyl-β-D-maltoside (DDM), digitonin, polysorbate 20, and polysorbate 80.

8. PROCESS, according to any of claims 1 to 7, **characterized in that** hydrolysis in an acidic environment being performed with an acid selected from the group consisting of acetic acid, hydrochloric acid, citric acid, lactic acid, and trifluoroacetic acid, preferably acetic acid or hydrochloric acid, more preferably acetic acid.

9. PROCESS, according to any of claims 2 to 8, **characterized in that** neutralization is by dialysis or performed with a basic solution, preferably a solution comprising sodium hydroxide and/or sodium bicarbonate, optionally in conjunction with a buffer solution comprising one or more of sodium bicarbonate buffer, phosphate-saline buffer, Dulbecco's phosphate-saline buffer, HEPES (4-(2-hydroxyethyl)-1-piperazineethanesulfonic acid), MES (2-(N-morpholino)ethanesulfonic acid), and MOPS (3-(N-morpholino)propanesulfonic acid).

10. PROCESS, according to any of claims 1 to 9, **characterized in that** the tissue or organ of origin is selected from the group consisting of adipose tissue, bone tissue, brain, cartilage tissue, colon, kidney, liver, lung, muscle tissue, myocardial tissue, pancreas, skin, spleen, stomach, vascular tissue, dental tissue, glandular tissue, such as the adrenal gland, the parathyroid gland, the thyroid gland, the pituitary gland, and the salivary gland, bone marrow tissue, tissue of the male reproductive system, such as the testicle, epididymis, prostate, seminal vesicle, and vas deferens, tissue of the female reproductive system, such as the vagina, cervix, endometrium, fallopian tube, ovary, and placenta, intestine, urinary bladder, and eye.

11. TISSUE-SPECIFIC HYDROGEL, **characterized in that** is produced by enzyme-free processes as defined in any of claims 3 to 10.

12. PROCESS FOR PRODUCING A PRODUCT DERIVED FROM TISSUE-SPECIFIC EXTRACELLULAR MATRIX SOLUTION, **characterized in that** comprises subjecting the tissue-specific extracellular matrix solution, produced by the enzyme-free process, as defined in any of claims 1 to 2, to one or more of the following chemical modifications:
(i) introduce acrylate derivatives (such as methacrylation);
(ii) introduce alkenes, for thiol-ene chemistry (such as thiol-norbornene);
(iii) introduce thiols, for thiol-ene chemistry or intermolecular disulfide bridges; and/or
(iv) introduce other chemical groups for bioorthogonal reactions (such as aldehyde and hydrazide).

13. PROCESS, according to claim 12, **characterized in that** additionally comprises cross-linking the modified tissue-specific extracellular matrix solution for transition to a hydrogel.

14. PRODUCT DERIVED FROM TISSUE-SPECIFIC EXTRACELLULAR MATRIX SOLUTION, **characterized in that** the product is the tissue-specific solution, produced by the enzyme-free process, as defined in any of claims 1, 2, 5, and 7 to 10, or the tissue-specific hydrogel obtained from it, as defined in claim 11, or produced by enzyme-free processes, as defined in any of claims 3 to 10, comprising one or more of the following chemical modifications:
(i) addition of acrylate derivatives (such as methacrylation);
(ii) addition of alkenes to thiol-ene chemistry (such as thiol-norbornene);
(iii) addition of thiols to thiol-ene chemistry or intermolecular disulfide bridges; and/or
(iv) addition of other chemical groups for bioorthogonal reactions (such as aldehyde and hydrazide).

15. PROCESS FOR PRODUCING A TISSUE-SPECIFIC SOLUBLE POWDER, **characterized in that** comprises subjecting the tissue-specific neutral extracellular matrix solution, produced by the enzyme-free process to produce said solution, as defined in any of claims 2, 5, and 7 to 10, or the tissue-specific hydrogel, as defined in claim 11, or produced by the enzyme-free processes, as defined in any of claims 3 to 10, or the product derived from the tissue-specific extracellular matrix solution, as defined in claim 14, or produced by the process to produce a product derived from the tissue-specific extracellular matrix solution, as defined in any of claims 12 to 13, to freeze-drying and spraying.

16. TISSUE-SPECIFIC SOLUBLE POWDER, **characterized in that** is obtained by freeze-drying and spraying of the tissue-specific neutral extracellular matrix solution, produced by the enzyme-free process to produce the solution, as defined in any of claims 2, 5, and 7 to 10, or of the tissue-specific hydrogel, as defined in claim 11, or produced by the enzyme-free process, as defined in any of claims 3 through 10, or of the product derived from the tissue-specific extracellular matrix solution, as defined in claim 14, or produced by the process to produce a product derived from the tissue-specific extracellular matrix solution, as defined in any of claims 12 through 13.

17. PROCESS FOR PRODUCING A TISSUE-SPECIFIC MEMBRANE, **characterized in that** comprises submitting the tissue-specific neutral extracellular matrix solution, produced by the enzyme-free process to produce said solution, as defined in any of claims 2, 5, and 7 to 10, or the tissue-specific hydrogel, as defined in claim 11, or produced by enzyme-free processes, as defined in any of claims 3 to 10, or the product derived from the tissue-specific extracellular matrix solution, as defined in claim 14, or produced by the process to produce a product derived from the tissue-specific extracellular matrix solution, as defined in any of claims 12 to 13, to dehydration.

18. TISSUE-SPECIFIC MEMBRANE, **characterized by** being obtained by dehydration of the tissue-specific neutral extracellular matrix solution, produced by the enzyme-free process to produce such solution, as defined in any of claims 2, 5, and 7 through 10, or of the tissue-specific hydrogel, as defined in claim 11, or produced by enzyme-free processes, as defined in any of claims 3 through 10, or from the product derived from the tissue-specific extracellular matrix solution, as defined in claim 14, or produced by the process to produce a product derived from the tissue-specific extracellular matrix solution, as defined in any of claims 12 through 13.

19. PROCESS TO PROVIDE BIOACTIVE COMPOUNDS FOR TISSUE REGENERATION, using the tissue-specific neutral extracellular matrix solution, produced by the enzyme-free process to produce said solution, as defined in any of claims 2, 5, and 7 to 10, the tissue-specific hydrogel, as defined in claim 11, or produced by enzyme-free processes, as defined in any of claims 3 to 10, or the product derived from the tissue-specific extracellular matrix solution, as defined in claim 14, or produced by the process to produce a product derived from the tissue-specific extracellular matrix solution, as defined in any of claims 12 to 13, **characterized in that** comprises:
(a) load the solution, the tissue-specific hydrogel, or the product derived from the tissue-specific extracellular matrix solution with the bioactive compound(s); and
(b) apply the solution, hydrogel, or product derived from the extracellular matrix solution loaded with the bioactive compound(s) on or within the target tissue to be regenerated.

20. PROCESS TO PROVIDE BIOACTIVE COMPOUNDS TO REGENERATE TISSUE, using the tissue-specific membrane, as defined in claim 18, or produced by the process to produce a tissue-specific membrane, as defined in claim 17, **characterized in that** comprises:
(a) load the tissue-specific neutral extracellular matrix solution, the tissue-specific hydrogel, or the tissue-specific extracellular matrix solution derivative product with the bioactive compound(s);
(b) dehydrate the solution, hydrogel, or product derived from the extracellular matrix solution loaded with the bioactive compound(s) to produce a membrane; and
(c) apply the membrane loaded with the bioactive compound(s) on the target tissue to be regenerated;
wherein steps (a) and (b) can occur in any order.

21. *IN VITRO* PROCESS TO PROVIDE BIOACTIVE COMPOUNDS THROUGH THE TISSUE-SPECIFIC HYDROGEL, as defined in claim 11, or produced by enzyme-free processes, as defined in any of claims 3 to 10, or through the product derived from the tissue-specific extracellular matrix solution, as defined in claim 14, or produced by the process to produce a product derived from the tissue-specific extracellular matrix solution, as defined in any of claims 12 to 13, wherein the hydrogel or the product derived from the extracellular matrix solution is for tissue regeneration, **characterized in that** comprises loading the tissue-specific hydrogel or the product derived from the tissue-specific extracellular matrix solution with the bioactive compound(s).

22. PROCESS, according to claim 21, **characterized in that** further dehydrates the hydrogel or the product derived from the extracellular matrix solution loaded with the bioactive compound(s) to produce a membrane.

23. PROCESS, according to any of claims 19 to 22, **characterized in that** the bioactive compound(s) is selected from the group consisting of:
- cells, preferably differentiated adult cells and stem cells; preferably wherein differentiated adult cells are cells derived primarily from endoderm, cells derived primarily from ectoderm, and cells derived primarily from mesoderm; and preferably wherein the stem cells are embryonic stem cells, induced pluripotent stem cells, and adult stem cells, or any combination thereof;
- Cell-secreted factors, preferably selected from the group consisting of growth factors, cytokines, chemokines, and micro-RNAs, or any combination thereof;
- vesicles secreted by cells, preferably selected from the group consisting of exosomes, microvesicles, apoptotic bodies, and ectosomes, or any combination thereof;
- nucleic acids, preferably selected from the group consisting of deoxyribonucleic acid (DNA), ribonucleic acid (RNA), messenger RNA (mRNA), transfer RNA (tRNA), ribosomal RNAs (rRNA), microRNAs (miRNA), small interfering RNAs (siRNA), piwi-interacting RNAs (piRNA), small nucleolar RNAs (snoRNAs), small nuclear RNAs (snRNA), extracellular RNAs (exRNA), Cajal body-specific small RNAs (scaRNA), long non-coding RNAs (IncRNA), doublestranded RNAs (dsRNA), and circular RNAs, or any combination thereof;
- medications;
- biological medical products, preferably selected from the group consisting of chimeric antigen receptor T cells (CAR T cells), monoclonal antibodies, fusion proteins, tumor necrosis factor α (TNFα), leukocyte surface glycoproteins, adhesion molecules, immunoglobulins, receptor targets, and viruses, or any combination thereof;
- metallic particles (such as silver nanoparticles), ceramic particles (such as hydroxyapatite), organic particles (such as nanoemulsions and polymeric nanoparticles), lipid particles (such as liposomes) and carbon nanomaterials (such as fullerenes, carbon nanotubes, graphene and graphene oxide), or any combinations thereof;
or any combination thereof.

24. USE OF NEUTRAL TISSUE-SPECIFIC EXTRACELLULAR MATRIX SOLUTION produced by the enzyme-free process to produce such solution as defined in any of claims 2, 5, and 7 to 10, or of tissue-specific hydrogel as defined in claim 11, or produced by enzyme-free processes as defined in any of claims 3 to 10, or the product derived from the tissue-specific extracellular matrix solution, as defined in claim 14, or produced by the process to produce a product derived from the tissue-specific extracellular matrix solution, as defined in any of claims 12 to 13, **characterized in that** is for the manufacture of a product.

25. USE, according to claim 24, **characterized in that** the product is for regenerating tissue, and wherein the tissue-specific neutral extracellular matrix solution, or the tissue-specific hydrogel or the product derived from the tissue-specific extracellular matrix solution is loaded with one or more bioactive compounds, wherein the one or more bioactive compounds are selected from the group consisting of cells, cell-secreted factors, cell-secreted vesicles, nucleic acids, drugs, biological medical products, metal particles (such as silver nanoparticles), ceramic particles (such as hydroxyapatite), organic particles (such as nanoemulsions and polymeric nanoparticles), lipid particles (such as liposomes) and carbon nanomaterials (such as fullerenes, carbon nanotubes, graphene, and graphene oxide), or a combination thereof.

26. USE, according to claim 24, **characterized in that** the product is selected from the group consisting of a tissue-specific soluble powder, a tissue-specific membrane, a loading carrier for bioactive compounds, such as for regenerating tissue, an additive ingredient for cell culture medium, a bioink for 3D bioprinting, and a surface coating solution for cell culture.

27. USE OF NEUTRAL TISSUE-SPECIFIC EXTRACELLULAR MATRIX SOLUTION produced by the enzyme-free process to produce such solution as defined in any of claims 2, 5, and 7 to 10, or of tissue-specific hydrogel as defined in claim 11, or produced by enzyme-free processes as defined in any of claims 3 to 10, or the product derived from the tissue-specific extracellular matrix solution, as defined in claim 14, or produced by the process to produce a product derived from the tissue-specific extracellular matrix solution, as defined in any of claims 12 to 13, **characterized in that** is as a tissue-specific soluble powder, as a tissue-specific membrane, as a loading carrier of bioactive compounds, such as to regenerate tissue and grow cells and/or cell aggregates, as an additive ingredient for cell culture media, as a bioink for 3D bioprinting, and as a surface coating solution for cell culture.

28. USE OF TISSUE-SPECIFIC EXTRACELLULAR MATRIX SOLUTION, produced by the enzyme-free process, as defined in any of claims 1, 2, 5, and 7 through 10, **characterized in that** is for the manufacture of derived products, wherein the products comprise the solution with one or more of the following chemical modifications:
(i) addition of acrylate derivatives (such as methacrylation);
(ii) addition of alkenes to thiol-ene chemistry (such as thiol-norbornene);
(iii) addition of thiols to thiol-ene chemistry or intermolecular disulfide bridges; and/or
(iv) addition of other chemical groups for bioorthogonal reactions (such as aldehyde and hydrazide).

29. ADDITIVE INGREDIENT FOR CELL CULTURE MEDIUM, **characterized in that** comprises tissue-specific soluble powder as defined in claim 16.

30. PROCESS FOR CULTIVATING CELLS WITHIN THE TISSUE-SPECIFIC HYDROGEL, as defined in claim 11, or produced by the enzyme-free processes, as defined in any of claims 3 to 10, or of the product derived from the tissue-specific extracellular matrix solution, as defined in claim 14, or produced by the process to produce a product derived from the tissue-specific extracellular matrix solution, as defined in any of claims 12 to 13, **characterized in that** the process comprises:
(a) load the tissue-specific neutral extracellular matrix solution, produced by the enzyme-free process to produce such solution, as defined in any of claims 2, 5, and 7 to 10, or the derived product of the tissue-specific extracellular matrix solution with the cells, at a cell concentration between 1 × 10³ and 1 × 10¹⁰ cells/mL;
(b) cross-linking the tissue-specific neutral extracellular matrix solution or the derived product of the tissue-specific extracellular matrix solution with the cells to produce a 3D cross-linked construct; and
(c) provide cells with growth conditions in such a way as to allow them to populate the 3D lattice construct to form mature functional tissue.

31. PROCESS FOR GROWING CELL AGGREGATES WITHIN TISSUE-SPECIFIC HYDROGEL, as defined in claim 11, or produced by enzyme-free processes, as defined in any of claims 3 to 10, or from the product derived from the tissue-specific extracellular matrix solution, as defined in claim 14, or produced by the process to produce a product derived from the tissue-specific extracellular matrix solution, as defined in any of claims 12 to 13, **characterized in that** the process comprises:
(a) load the tissue-specific neutral extracellular matrix solution produced by the enzyme-free process to produce said solution as defined in any of claims 2, 5, and 7 to 10, or the product derived from the tissue-specific extracellular matrix solution with the cell aggregates, such as spheroids, organoids, and cell monolayers;
(b) cross-linking the tissue-specific neutral extracellular matrix solution or the derived product of the tissue-specific extracellular matrix solution with the cell aggregates to produce a 3D cross-linked construct; and
(c) provide cell aggregates with conditions for growth or maintenance.

32. PROCESS FOR BIOPRINTING 3D BIOINKS, comprising cells or cell aggregates within the tissue-specific hydrogel as defined in claim 11, or produced by the enzyme-free processes as defined in any of claims 3 to 10, or from the product derived from the tissue-specific extracellular matrix solution as defined in claim 14, or produced by the process to produce a product derived from the tissue-specific extracellular matrix solution, as defined in any of claims 12 to 13, or from the tissue-specific extracellular matrix solution, produced by the enzyme-free process to produce a tissue-specific extracellular matrix solution, as defined in any of claims 2 and 7 to 10, **characterized in that** the process comprises:
(a) load cells or cell aggregates into the tissue-specific hydrogel or the product derived from the tissue-specific extracellular matrix solution, or into the tissue-specific neutral extracellular matrix solution, or in combinations of such hydrogels or products or solutions with other biomaterials;
(b) load the mixture described in item (a) into the printing chamber of a 3D bioprinter;
(c) perform the 3D printing steps, according to the mechanism of action of the 3D bioprinter;
(d) reticular to stabilize the bioprinted 3D construction; and
(e) provide cells with growth conditions in such a way as to allow them to populate the bioprinted 3D construct to form a mature functional tissue.

33. PROCESS FOR 3D BIOPRINTING, FROM A TISSUE-SPECIFIC EXTRACELLULAR MATRIX SOLUTION, **CHARACTERIZED IN THAT** COMPRISES:
produce a tissue-specific extracellular matrix solution through steps (a) and (b) of the enzyme-free process as defined in any of claims 1, 5, 7, 8, and 10;
(c) load the hydrolyzed extracellular matrix solution from step (b) into the printing chamber of a 3D bioprinter;
(d) perform the 3D printing steps, according to the mechanism of action of the 3D bioprinter; and
(e) reticulate to stabilize the bioprinted 3D construction.

34. PROCESS, according to claim 33, **characterized in that**, between steps (b) and (c), proceeds with the step of neutralizing the pH of the hydrolyzed extracellular matrix solution from step (b) to obtain a tissue-specific neutral extracellular matrix solution.

35. PROCESS, according to claims 33, **characterized in that** proceeds with the pH neutralization step of the hydrolyzed extracellular matrix solution during or after the bioprinting of step (d) or after crosslinking to stabilize the bioprinted 3D construction of step (e).

36. PROCESS, according to any of the claims 33 to 35, **characterized in that**, between steps (b) and (c), proceeds with the stage of incorporation of biomaterials and/or bioactive compounds.

37. PROCESS, according to any of claims 32 to 36, **characterized in that** the 3D bioprinter printing chamber comprises a syringe, a Petri dish, a flask, or another 3D bioprinting chamber.

38. PROCESS, according to any of claims 32 to 37, **characterized in that** the 3D bioprinter is an extrusion-based bioprinter, a stereolithography-based bioprinter, an inkjet-based bioprinter, a laser-based bioprinter, a tomography-based bioprinter, an acoustics-based bioprinter or an electrospinning-based bioprinter.

39. PROCESS, according to any of claims 3, 4 to 10, 13, 30 to 32, and 38, **characterized in that** crosslinking preferably comprises one or more of the following: physical crosslinking, such as by changing temperature and/or pH, and adding ions to promote ionic interaction; and chemical crosslinking, such as a reaction to obtain covalent bonds, such as by exposure to light, including visible light, ultraviolet light, and infrared light.

40. KIT, **characterized in that** comprises the tissue-specific extracellular matrix solution, produced by the enzyme-free process, as defined in claim 1, or the tissue-specific soluble powder, as defined in claim 16, or produced by the process to produce said powder, as defined in claim 15, or the product derived from the tissue-specific extracellular matrix solution, as defined in claim 14, or produced by the process to produce a product derived from the tissue-specific extracellular matrix solution, as defined in any of claims 12 to 13.
